# EUROPEAN PATENT SPECIFICATION

(11) **EP 3 119 278 B1**
(45) Date of publication and mention of the grant of the patent: **23.02.2022**
(21) Application number: 15764503.7
(22) Date of filing: 20.03.2015
(51) Int. Cl.: A61B 5/08, A61B 5/097

(54) **SELECTION, SEGMENTATION AND ANALYSIS OF EXHALED BREATH FOR AIRWAY DISORDERS ASSESSMENT**
AUSWAHL, SEGMENTIERUNG UND ANALYSE VON AUSGEATMETEM ATEM ZUR ATEMWEGSERKRANKUNGSBEURTEILUNG
SÉLECTION, SEGMENTATION ET ANALYSE DE L'AIR EXPIRÉ POUR L'ÉVALUATION DE TROUBLES DES VOIES RESPIRATOIRES

(30) Priority: 20.03.2014 US 201461968290 P
(43) Date of publication of application: 25.01.2017
(73) Proprietor: Capnia, Inc., Foster City, CA 94404 (US)
(72) Inventor: WONDKA, Anthony, D., San Ramon, CA 94582 (US); BHATNAGAR, Anish, Redwood City, CA 94065 (US)
(74) Representative: Cooley (UK) LLP
(86) International application number: PCT/US2015/021852
(87) International publication number: WO 2015/143384

(56) References cited:
- WO-A1-2011/101776
- US-A1- 2006 200 037
- US-A1- 2010 317 986
- US-A1- 2011 196 295
- US-A1- 2012 310 104
- US-A1- 2013 165 806
- US-A1- 2013 165 806
- US-A1- 2013 267 862

## Description

### TECHNICAL FIELD

Described herein are devices and methods for the analysis of breath exhalant for diagnostic purposes. More specifically, devices and methods are described for sampling and analyzing a relevant portion of the breathing cycle, for example exhaled gas stemming from the lung airways, from a person's breath that may be used to correlate the gas analysis to an underlying physiologic condition for diagnostic purposes.

### BACKGROUND

Certain metabolites and chemicals produced in or entering the body and blood stream are excreted in the exhaled breath. The level in the body or blood stream may be determined by measuring it in the breath. Often, certain diseases cause abnormal levels of a certain analyte(s), in which case a correlation typically exists between the concentration level of the analyte being measured, and the degree of the underlying disease. The analyte being measured can be a non-gaseous substance, such as particulates and other chemicals, or a gaseous substance. For example, breath NO levels may be measured to detect and monitor underlying disorders such as disorders of the lung airways. Other analytes found in the breath may similarly be measured to assess diseases or conditions of other organs and physiological systems. Breath NO measurements, for example for asthma assessment, is used as an example throughout this disclosure, however it should be noted that other analytes and other clinical conditions are also contemplated, such as measuring other blood gases such as O, CO and CO2, ph of the exhaled breath, bacteria, proteins, acids, VOC's, aldehydes, ketones, blood alcohol, ammonia, H2, acetone, urea, or methane for assessing a variety of conditions such as respiratory problems, digestive system problems, kidney problems, liver problems, endocrine problems, sleep disorders, blood chemistry problems, pancreatic problems, psychological problems, orthopedic problems, cardiovascular problems, problems with other organ systems, systemic problems, infections, drug use, hematological problems, cancer, or genetic problems or for sports medicine, or for homeland security.

The measurement of breath NO is widely reported in the literature and prior art as a method to test for asthma. In asthma there is an inflammation process of the underlying tissues of the middle and lower lung airways. This inflammation generates nitric monoxide gas, or NO, which diffuses into the airways of the lung, and is then exhaled during breathing. In asthma assessment therefore, the NO specifically coming from the lung airways is of interest. The NO in alveolar gas may not be of interest in asthma assessment, and nasal airway gas which can be elevated for other reasons, may also not be of interest. NO gas coming from the stomach can be dismissed when performing breath NO asthma assessments because of the esophageal sphincter, if the patient's recent ingestion history is known. When breath NO monitoring is used clinically, elevated levels of NO can be indicative of the onset of an asthma attack. In addition, NO monitoring can be useful in assessing the response to asthma medication and treatment, progress toward being cured, and compliance to asthma treatment over time.

As reported in the medical literature, the concentration of nitric oxide in exhaled breath is expiratory flow rate dependent. If the expiratory flow rate is faster than normal, the exhaled gas contains less NO because the alveolar gas reaches the airways more quickly and there is less time for the NO to diffuse into the lower airways of the lung (reference Silkoff, Am J Respir Crit Care Med 1997;155:260-267). If the expiratory flow rate is slower than normal, the exhaled gas may conversely contain more NO. Because of these breathing dynamics and diffusion rules, and in order to obtain an accurate NO measurement, and a measurement that is standardized across different instruments, locations and clinical practices, the medical community has adopted standard guidelines related to expiratory flow rate. The recommended flow rate is a certain flow rate that the patient must maintain for a period of time during expiration (reference Am J Respir Crit Care Med Vol 171.pp 912-930, 2005). Multiple tests are recommended. In other clinical scenarios, it might be useful for the patient to submit two exhaled samples at different expiratory flow rates; one with a fast expiratory flow rate and one with a slow expiratory flow rate, and the difference in FENO ("fraction of exhaled NO") may be clinically revealing for certain situations. Nonetheless, whether conforming to the recommended flow rate, or if doing a two flow rate test, the standard guidelines may require a certain flow rate needs to be followed by the patient. Typically, a flow or pressure sensor is included in the mouthpiece which is used to instruct to the patient in real time how strong to exhale in order to maintain the desired flow rate for the necessary period of time.

While these techniques are suitable to the adult, adolescent and older pediatric population when they are cognizant, not surprisingly younger pediatrics and non-cognizant patients, or non-cooperative patients, cannot reliably submit a breath sample at the required expiratory flow rate, even despite practice and despite information, instructions and coaching provided by the device or clinician. Some percentage of tests with test patients, for example 20% of the attempted tests, may possibly conform to the required flow rate and yield a valid sample and the other non-conforming tests can simply be automatically discarded by the instrument using the appropriate algorithms. However, this hit or miss situation is frustrating to the users, sheds suspicion on the results because fatigue may affect the measurement, and for this and other reasons breath NO assessments in these populations has not been accepted or adopted in actual clinical practice. Indeed, for at least these reasons the USFDA approved labeling for all of the FDA approved breath NO analyzers state a minimum age requirement of 7 or 8 years old (ref. K072816, K101034, K021133, K073265, K083617).

US2006/200037 discloses a system and method for selectively collecting exhaled air. The system includes a sensor that can be configured for detecting a flow of exhaled air from a person. A control system can be responsive to the sensor. The control system can be configured for generating a gate signal when at least one characteristic of the flow of exhaled air satisfies one or more predetermined flow criteria. A valve responsive to the gate signal can be configured for selectively gating only a predetermined portion of the flow of exhaled air to an air sample chamber.

In US2013/165806, apparatuses are described to accurately determine a gas concentration of a sample of a patient's breath.

### BRIEF SUMMARY

The subject-matter of the present invention is defined by the claims 1 to 15.

Therefore, there appears to be an unmet need for reliable and accurate collection and analysis of exhaled breath NO for non-cognizant and younger pediatric patients. In the present disclosure, systems and methods are described which overcome the obstacles associated with reliable and accurate breath sample collection for FENO measurements in non-cognizant, non-cooperative and young pediatric populations. And again, while the primary example used in this disclosure relates to breath NO measurements for asthma assessment, the same principles apply to measuring other analytes in other parts of the bronchopulmonary tree for assessing other clinical conditions.

In a variation, the patient may be instructed to breathe normally. This may provide an alternative requiring a patient to follow expiratory flow rate instructions. Instead of breathing into a mouthpiece as in the case of prior art systems, a nasal cannula may be used to draw the sample from the patient and the patient simply breathes normally. Breathing into a mouthpiece can be obtrusive and typically causes young children to breathe abnormally. In contrast, breathing with a non-obtrusive nasal cannula prong inserted into a nares may allow a young child to breathe without any encumbrance. The nasal cannula may be coupled to a vacuum source inside the instrument to automatically draw breathing gas from the patient, without the patient having to participate. Other types of patient interfaces are also contemplated and can be used to collect the sample. For example a non-obtrusive mouthpiece also coupled to a vacuum source can be used with which the patient can feel like they are breathing naturally. In addition, a face mask may be used which covers both the oral and nasal cavity, with an inspiratory valve and expiratory valve, with the instrument's sampling port coupled to the expiratory valve outlet. Or, alternatively, the patient interface can be a nasal mask which seals around the nose, rather than a nasal cannula. Regardless of the interface, they may be designed differently from interfaces described in the prior art in that interfaces described herein may have reduced deadspace such that there may be a rapid complete exchange of mask gas volume as air is being exhaled. When using a nasal interface to collect the exhaled gas sample, a mouthpiece may be used which discourages breathing through the mouth so that the patient is breathing predominantly or completely through the nares. When using an oral interface to collect the exhaled gas sample, a nose clip may be used to discourage breathing through the nose.

In a variation, the instrument may provide a calming audible and/or visual metronome to the patient so that the patient breathes at a respiratory rate of the metronome. The metronome rate may be chosen to be a rate normal for the specific patient being tested, in order to get an accurate FENO measurement. This rate may be automatically determined based on certain patient criteria such as age, height, weight or gender, or may be selected by the clinician prior to the test, or a combination. The patient may be given an acclimation period of breathing according to the metronome, that a period suitable to establish steady state conditions of gas concentrations throughout the pulmonary tree is used. A breathing pattern sensor may used to verify that the actual breathing pattern complied or complies with the required acclimation requirements in order for the test to continue. After the acclimation period is complete and verified as valid, the instrument may begin to collect exhaled gas for measurement of FENO. In addition to using respiratory rate as a guideline for patient breathing, other breathing signals can be used such as breathing pressure, breathing flow rate, chest excursions, breath sounds, or Capnometer.

In some variations, one of two techniques may be used: a single breath measurement of NO, and a multiple breath measurement of NO. The former may be described throughout most of the subsequent description however both techniques apply. In the case of acquiring samples from multiple breaths, the variations described in Capnia's provisional patent application 61/872,415 may apply, and the content of that application is incorporated by reference herein in its entirety.

In a variation, after the acclimation period, typically a valid breath is identified using a breathing sensor and using breath pattern criteria, in order to limit measuring NO in an abnormal breath. The valid breath may be that which will yield a physiologically valid NO sample. Breaths may be classified based on the breathing sensor data, for example as normal tidal volume breaths, partial breaths, deep breaths, sigh breaths, stacked breaths, breaths after apnea, fast breaths, slow breaths, normal duration breaths, etc. In order for a breath to qualify as containing a valid gas sample, based on the breathing sensor data, the instrument may verify that the breath conforms to the expected respiratory rate, otherwise, the measured NO may be artificially high or low. In addition, the breath may be required to meet other breathing signal amplitude and duration requirements before sampling. Also, the breaths before the sampled breath may also be required to conform to the expected respiratory rate and other requirements in order to insure a stable breathing pattern prior to the sample being collected, otherwise the gases in the bronchopulmonary tree may fall out of equilibrium. If the breathing pattern doesn't fall within the expected respiratory rate range, the test may continue until the patient is breathing within this range. Eventually, even if the patient's respiratory rate ("RR") is bothered by the minimal invasiveness of the test, the patient may eventually calm down and breathe at a normal rate because of the respiratory control mechanism of the brain. In addition to using respiratory rate as a criteria for a defining a valid target breath, other breathing signals can be used such as breathing pressure, breathing flow rate, chest excursions, breath sounds, or Capnometer. In the case of defining and selecting a valid breath for analysis, the variations described in Capnia's earlier patent application 14/150,625 may apply and the content of that application is incorporated by reference herein in its entirety.

In a variation, once a breath is targeted, algorithms may be used to separate out a sample of expiratory gas from the middle and/or lower airways and to discard gas from nasal cavity, trachea and alveolar areas. Separating the expiratory gases may be advantageous to prevent contamination of the sample. For example, the concentration of NO gas from the nasal cavity can be higher than airway NO and can be highly variable and therefore will adversely affect the accuracy and repeatability of the assessment. An expiratory phase sensor that measures the exhalation of the patient may be used to identify different portions of the expiratory phase, and the instrument uses these identified portions to separate out the desired portion from the undesired portions. Some examples of sensors include capnometers, airway pressure sensors, flow sensors, chest excursion sensors, esophageal sensors, respiratory drive sensors, and breath sound sensors. The signal can be measured in line with the expiratory gas collection conduit, or can be a separate measurement channel or connection to the patient, depending on the sensor used, or a combination. The breathing signal can be differentiated, transformed or otherwise converted in order to better identify transition points during the expiratory phase that correspond to different sections of the bronchopulmonary tree. The information collected from the sensor regarding the identification of different portions of the expiratory phase may be correlated to a location of each expiratory phase portion of gas collected by the instrument. For example, the beginning of exhalation can be identified by a sudden positive airway pressure at a time t(a). Because the speed of travel of the gas through the system is known, and the various lengths of conduit within the system is known, the location throughout the system of the gas corresponding to time t(a) is known. The sensor used to identify and segment the different portions of the expiratory phase may be the same sensor used to qualify and disqualify breaths as suitable or valid target breaths from which to acquire a sample, however they can also be different sensors.

In a variation, after collection of a valid airway gas sample, the sample is measured for airway NO level (aNO). The measurement of aNO can happen on-board or off-board the instrument. In on-board systems, the measurement can be made instantaneously, semi-instantaneously, in substantially real time during collection of the sample, or can be performed after a delay in obtaining the sample, depending on the type of NO sensor technology deployed in the instrument. If the NO sensor is an instantaneous sensor, or near-instantaneous, the NO sensor can be both the breathing signal sensor and NO sensor. The system's pneumatics for collecting the sample can vary, and some of these forms of collection instruments have been further described in Capnia's provisional patent application 61/872,514 the content of which is incorporated by reference herein in its entirety.

In a variation, the patient may be prompted to breathe fast for a while during which time a sample is collected, and then slow for a while for collection of a second sample. This may permit the instrument to perform an aNO comparison between fast and slow breathing which is sometimes useful in certain clinical situations as explained earlier. Again, the metronome may guide the patient to breathe at the desired respiratory rate. The period of slow and fast breathing for example can be two minutes each, with an appropriate rest period in between so that the gas gradients in the lung can reestablish equilibrium in between.

Similarly, for certain other diagnostic situations, it may be beneficial or necessary to increase the sensitivity of the test which can sometimes be done by provoking the patient in some other way. For example the patient can be exposed to a substance injected into the body or breathed into the lungs. Body position may be taken into consideration as well, and some tests may be better performed with the patient sitting, while others may be better with the patient lying down, due to the effect that body position has on gas gradients within the pulmonary tree and respiration and ventilation. The ambient surroundings often need to be taken into consideration to account for background gases that could be additive to the breath gases. This can be done by performing an ambient measurement and making the appropriate correction. In other situations, environmental vapors, gases and particles which may interfere with the test may be dealt with using filters, test compartments, acclimation periods, correction factors, warnings and the like.

A minimum volume may be needed by the NO sensor in order to satisfy the sensor's signal response characteristics. In a seventh variation, the flow rate of the sampling system may be set to draw in the minimum volume of the patient's exhaled breath. For example, the following patient parameters and sensor requirements may be used for a certain test: (1) the patient is a 4 year old with a 200ml tidal volume, with a 15 breaths per minute normal respiratory rate and with 2 second expiratory time during normal tidal volume breathing, (2) a 5ml sample is required to satisfy the NO sensor's signal response characteristics to register an accurate measurement, (3) the part of the lung of interest for the test is the middle 1/5th of the overall expiratory flow. Based on these considerations, 5ml/(2sec^{∗}1/5) is the required sampling flow rate of the system, equating to 12.5ml/sec or 750ml/min which is about 6% of the patient's expiratory flow and also about 6% of the patient's inspiratory flow. In many clinical situations it may be desirable to keep the sampling flow rate to below 10% of the patients inspiratory flow in order to not affect the breathing, and to below 10% of the expiratory flow in order to prevent generating NEEP. Therefore the overall system requirements including (a) the expected patient breathing pattern, (b) the sensor signal response, (c) the gas sample size and (d) the sampling flow rate, may be taken into account in the system's design.

In a variation applied to measuring aNO for asthma assessment, it is described that looking at the middle 1/5^{th} of the expiratory gas is valid. However for some other asthma assessments and for other diagnostic tests, exhaled gas may be into any number of sections, such as six to ten or more partitions, is also contemplated.

In another variation it is contemplated that in asthma, a specific level of lung airways may be prone to the inflammatory response arising from a certain exogenous or endogenous irritant leading to an exacerbation. And it is contemplated that different genotypes of the disease affect different areas of the lung airways. For example inflammation of the segmental bronchi may correlate to a certain type of asthma, or a certain type of irritant causing an attack. And similarly, inflammation of the lower airways such as the 6^{th} -8^{th} generation of branches, may correlate to yet another type of asthma or a different irritant. The variations described herein may also be used to determine which section of the lung the NO is highest, or to create a NO mapping or inflammation mapping of the bronchopulmonary tree, and determine the areas most affected by the inflammation. Invasive techniques to map the inflammation throughout the lung are likely possible, but very invasive, expensive and risky. In some variations, this information may be obtained completely non-invasively and without risk to the patient. This information may then be even more useful in diagnosis and also useful to guide treatment. This information can help the clinician determine the optimal treatment and even a cure. For example, for bronchoplasty treatment, the measurements obtained from variations herein can help inform the interventional pulmonologist on which airways in the lung may need to be treated, and can therefore optimize treatment, stage treatments over time, and avoid over-treating or undertreating. The mapping diagnosis can be performed in advance of the treatment or at the same time as the treatment.

In a first variation, an apparatus for analyzing a breath analyte comprises: a breathing sensor to measure a breathing pattern parameter; a breath sampling system comprising a breath gas collection conduit; a first processor to (i) establish breathing pattern parameter criteria wherein the criteria delineates between a physiologically representative breath and a physiologically non-representative breath, and (ii) to determine if the exhaled gas from a breath should be sampled for analysis based on a comparison of a breathing parameter criteria value to the measured breathing pattern parameter; an analyzer to compositionally analyze at least one section of the breath gas; and a controller to channel the at least one section of breath gas to the gas analyzer.

In a second variation, the apparatus of the first variation further comprises: a second processor executing a partitioning algorithm to divide the breath gas in the gas collection conduit into discrete sections based on data from the breathing sensor, the discrete sections divided so that at least one section represents a physiological section of the bronchopulmonary tree desired to be analyzed; and a third processor executing a locating algorithm to locate the position of at least one part of the desired physiological section of the breath gas in the gas collection conduit.

In a third variation, the partitioning algorithm partitions the breath gas as the exhaled section of the breath gas is measured by the breathing sensor. In a fourth variation, the partitioning algorithm partitions the breath gas after the breathing sensor completes the measurement of at least a portion of the exhaled section of the breath gas. In a fifth variation, the desired physiological section represents a section of the bronchopulmonary tree between the trachea and the alveolii. In a sixth variation, the desired physiological section represents a section of the bronchopulmonary tree selected from the group of: the nasal airway, the trachea, the main stem bronchii, the segmental bronchii, the conducting airways, the respiratory airways, the alveoli. In a seventh variation, the discrete sections comprise at least three sections, and wherein a middle section is the desired physiological section. In an eighth variation, the discrete sections comprises at least N number of sections, wherein the volume of gas in a section of 1/Nth is equal to or greater than the volume required by the gas analyzer for a measurement of the analyte. In a ninth variation, the partitioning algorithm partitions the exhaled gas by measuring a duration of at least a part of the exhalation phase and dividing the duration of that part into multiple time sections. In a tenth variation, the partitioning algorithm partitions the exhaled gas by detecting characteristics in the breathing sensor signal, the characteristics selected from the group: a zero signal amplitude, a peak signal amplitude, a crossing of zero from a negative value to a positive value, a crossing of zero from a positive value to a negative value, a plateau in the signal amplitude, a change in slope of the signal amplitude, a zero of the differential of the signal, a peak of the differential of the signal, a zero of the second differential of the signal, a peak of the second differential of the signal, a zero of a transform of the signal, a peak of a transform of the signal. In an eleventh variation, the at least one part of the desired section is selected from the group: a beginning of the section, an end of the section, a mid-point of the section.

In a twelfth variation, the breathing sensor of the first variation is selected from the group: CO2 sensor, pressure sensor, flow sensor, acoustical sensor, chest movement sensor, gas composition sensor, the analyte analyzer. In a thirteenth variation, the breathing sensor and analyte analyzer are the same sensor. In a fourteenth variation, the breath analyte is nitric oxide. In a fifteenth variation, the breath analyte includes but is not limited to the group of: NO, CO, H2, ammonia, humidity PH, bacteria, CO2, O2, VOC's, blood alcohol, urea, acetone, methane, acids, proteins, or combinations thereof. In a sixteenth variation, the analyzer is selected from the group of: optical, chemical, electrical, electrochemical, chemiiluminesence, chromatographical, opto-electrical, opto-chemical.

In a seventeenth variation, the breath sampling system comprises a cannula with a machine end connectable to the apparatus and a distal end adapted to be disposed in the path of a patient airway, and a flow source connectable to the cannula. In an eighteenth variation, the system of the first variation further comprises a fourth processor executing an algorithm to notify the patient to breathe at a desired frequency. In a nineteenth variation, the system of the first variation further comprises a fifth processor executing an algorithm to notify the patient to breathe at a desired frequency, an algorithm to delay the collection of the breath gas for analysis for a period of time while the patient is breathing at the desired frequency, and an algorithm to verify that the patient is breathing at the desired frequency. In a twentieth variation, the system of the first variation further comprises a sixth processor executing an algorithm to measure the analyte in at least two sections of the exhaled gas and to correct the level of analyte measured in a physiologically valid section with the analyte measured in another section. In a twenty-first variation, the system of the first variation further comprises a seventh processor executing an algorithm to measure the analyte in multiple breaths for determining a final compositional value.

In a twenty-second variation, a method for analyzing a breath analyte, comprises: collecting at least one portion of a breath exhalation into a conduit; measuring an exhalation signal of the breath using a breathing sensor; and determining if a breath is physiologically valid for analysis of a breath analyte by comparison of the measured exhalation signal to a criteria and if determined valid, analyze at least one section of the breath exhalation for the breath analyte using an analyzer.

In a twenty-third variation, the method of the twenty-second variation further comprises partitioning the breath gas in the conduit into discrete sections based on data from the breathing sensor so that at least one section represents a desired physiological section of the bronchopulmonary tree; locating the position of at least one part of the desired physiological section of the breath gas in the conduit based on data from the breathing sensor; and channeling the at least one part of the desired physiological section of gas to the analyzer.

In a twenty-fourth variation, the breath gas is partitioned as the breath gas is being measured by the breathing sensor. In a twenty-fifth variation, the breath gas is partitioned after the breathing sensor completes the measurement of at least a portion of the exhaled section of the breath gas. In a twenty-sixth variation, the partitioning partitions a section of gas from the bronchopulmonary tree between the trachea and the alveolii. In a twenty-seventh variation, the partitioning partitions a section of gas from the bronchopulmonary tree from a section selected from the group of: the nasal airway, the trachea, the main stem bronchii, the segmental bronchii, the conducting airways, the respiratory airways, the alveoli. In a twenty-eighth variation, the partitioning partitions a section of gas from into at least three sections, wherein a middle section is the desired physiological section. In a twenty-ninth variation, the gas is partitioned into at least N number of sections, wherein the volume of gas in a section of 1/Nth is equal to or greater than the volume required by the gas analyzer for a measurement of the analyte. In a thirtieth variation, the gas is partitioned by measuring the duration of at least a part of the exhalation phase and dividing the duration of that part into multiple time sections. In a thirty-first variation, the gas is partitioned by detecting and using characteristics in the breathing sensor signal, the characteristics selected from the group: a zero signal amplitude, a peak signal amplitude, a crossing of zero from a negative value to a positive value, a crossing of zero from a positive value to a negative value, a plateau in the signal amplitude, a change in slope of the signal amplitude, a zero of the differential of the signal, a peak of the differential of the signal, a zero of the second differential of the signal, a peak of the second differential of the signal, a zero of a transform of the signal, a peak of a transform of the signal. In a thirty-second variation, the gas is located by locating at least one part of the desired section which is selected from the group: a beginning of the section, an end of the section, a mid-point of the section

In a thirty-third variation, the method of the twenty-second variation further comprises using a breathing sensor selected from the group: CO2 sensor, pressure sensor, flow sensor, acoustical sensor, chest movement sensor, gas composition sensor, the analyte analyzer. In a thirty-fourth variation, the breathing sensor and analyte analyzer are the same sensor. In a thirty- fifth variation, the analysis is of nitric oxide. In a thirty-sixth variation, the analyzer analyzes at least one analyte selected from the group of but not limited to: NO, CO, H2, ammonia, humidity PH, bacteria, CO2, O2, VOC's, blood alcohol, urea, acetone, methane, acids, proteins, or combinations thereof. In a thirty-seventh variation, the analyzer is selected from the group of: optical, chemical, electrical, electrochemical, chemiiluminesence, chromatographical, opto-electrical, opto-chemical analyzers. In a thirty-eighth variation, the breath sample is collected using a nasal cannula coupled to an airway on one end and the apparatus on the other end, and using a vacuum source. In a thirty-ninth variation, the patient is instructed by the apparatus to breathe at a desired frequency.

In a fortieth variation, the method further comprises (i) the apparatus instructs the patient to breathe at a desired frequency, (ii) delaying the collection of the breath gas for analysis for a period of time while the patient is breathing at the desired frequency, and (iii) verifying that the patient is breathing at the desired frequency. In a forty-first variation, the method further comprises measuring the analyte in at least two sections of the exhaled gas and correcting the level of analyte measured in a physiologically valid section with the analyte measured in another section. In a forty-second variation, the method further comprises measuring the analyte in multiple breaths for determining a final compositional value

In a forty-third variation, a method of analyzing breath exhalant comprises: measuring a CO2 level of a breath expiratory phase; opening a valve to a conduit when the CO2 level goes from substantially zero to positive; measuring a pressure of the breath expiratory phase; closing the valve to the conduit when a first derivative of the pressure is zero; and analyzing gas flowing through in the conduit.

In a forty-forth variation, a system that analyzes breath exhalant comprising: a first conduit to collect the gas; a second conduit to analyze the gas; an inlet valve on the second conduit; a capnometer to measure a CO2 level of a breath expiratory phase, wherein the inlet valve opens when a the CO2 level goes from substantially zero to positive; a pressure sensor to measure the pressure of the breath expiratory phase, wherein the inlet valve closes when a first derivative of the pressure is zero; and an analyzer to measure gas traveling in the second conduit.

### BRIEF DESCRIPTION OF THE DRAWINGS

Figure 1 describes a schematic overview of a breath analysis apparatus with a sample tube, bypass tube and sample push tube, in accordance with a variation.
Figure 2 describes a schematic overview of an alternative breath analysis apparatus with a sample pathway and bypass pathway, in accordance with a variation.
Figure 3 describes a schematic overview of an alternative breath analysis apparatus with off board sample analysis, in accordance with a variation.
Figure 4 describes a schematic overview of an alternative breath analysis apparatus with a sample pathway and bypass pathway, in accordance with a variation.
Figure 5 describes a schematic overview of an alternative breath analysis apparatus with a parallel channel to the sample collection channel for breathing signal measurement, in accordance with a variation.
Figure 6 describes a schematic overview of an alternative breath analysis apparatus with a fast responding compositional sensor, in accordance with a variation.
Figure 7 describes a schematic overview of an alternative breath analysis apparatus in which the patient passively breathes into the instrument for sample collection, in accordance with a variation.
Figure 8 graphically describes a Capnometer signal, airway pressure signal and a flow sensor signal of a breath, in accordance with a variation.
Figure 9 graphically describes different partitions of an expiratory cycle using CO2 and airway pressure sensor signals and including a theoretical NO tracing, in accordance with a variation.
Figure 10 graphically describes a sequence of a test using a Capnometer signal tracing, in accordance with a variation.
Figure 11 graphically describes the breathing sensor signals of the breath bn from Figure 10, in accordance with a variation.
Figure 12a graphically describes the breathing sensor signals of Figure 11 with a flipped time scale, in accordance with a variation.
Figure 12b schematically describes the pneumatics of the apparatus with the expiratory gas aligned with the graphic in Figure 12a, in accordance with a variation.
Figure 13a graphically describes the breath bn shown in Figure 12a with the inspiratory phase shown for breath bn and breath bn+1, in accordance with a variation.
Figure 13b describes a pneumatic schematic of the apparatus with gas sample from the expiratory phase of breath bn in the sample tube 18, with the schematic aligned with the graphic in Figure 13a, in accordance with a variation.
Figure 14 graphically describes a test sequence in which samples are obtained and analyzed from different types of breathing patterns for enhanced sensitivity and specificity, in accordance with a variation.
Figure 15 graphically describes a gradient of airway NO gas corresponding to a certain genotype of asthma or a type of airway inflammation triggered by a certain irritant, in accordance with a variation.
Figure 16 describes a nasal cannula patient interface for sample collection, in accordance with a variation.
Figure 17 describes a nasal mask cannula patient interface for sample collection, in accordance with a variation.
Figure 18 describes a oral mask patient interface for sample collection, in accordance with a variation.
Figure 19 describes a face mask patient interface for sample collection, in accordance with a variation.

### DETAILED DESCRIPTION

Described here are devices and methods for obtaining a gas sample for analysis especially from young children, or non-cognizant or non-compliant patients. In particular obtaining an accurate and reliable and repeatable measurement is described by automatically collecting the sample, collecting the sample in a manner that does not make the patient's breathing abnormal or invalid for the test, partitioning the expiratory gases into different partitions corresponding to different sections of the pulmonary tree in the lung, isolating a expiratory gas partition that is physiologically valid for the analysis of interest, and measuring the sample for the analyte in question. In one variation, a collection of exhaled gas is taken from a physiologically valid breath of a young child, isolating gas from the middle portion of the exhaled gas representing the gas from the middle and lower airways, and measuring that portion of gas for NO for the assessment of asthma. In the variations shown, for exemplary purposes, NO gas middle airway measurements are described, and the patient's breath sample is shown to be drawn into the instrument from the patient by application of vacuum. However this disclosure also applies to measurement of other sections of the expiratory cycle, other breath gases, for other diagnostic purposes, and patients breathing into the instrument in order for the instrument to collect the breath sample.

In some variations, one or more breathing parameters may be measured to identify the different constituent portions of a breath and the respective time periods, and a pneumatic system may be used for capturing the portion of exhaled breath in a sampling tube using the identified time period. In some variations, one or more valves and/or flow control mechanisms, such as a vacuum pump for example, may be used to regulate the flow rate of gas drawn into the sampling tube. In some variations, the captured portion of breath may be analyzed for indications of a patient's physiological state.

Measured breathing parameters may include one or more of carbon dioxide, oxygen, airway pressure, airway temperature, breath flow rate, and chest impedance, diaphragmatic movement or innervation, breath sounds, or breath vibrations. Identifying the time period of a portion of a breath may include identifying substantially the start and termination of that time period.

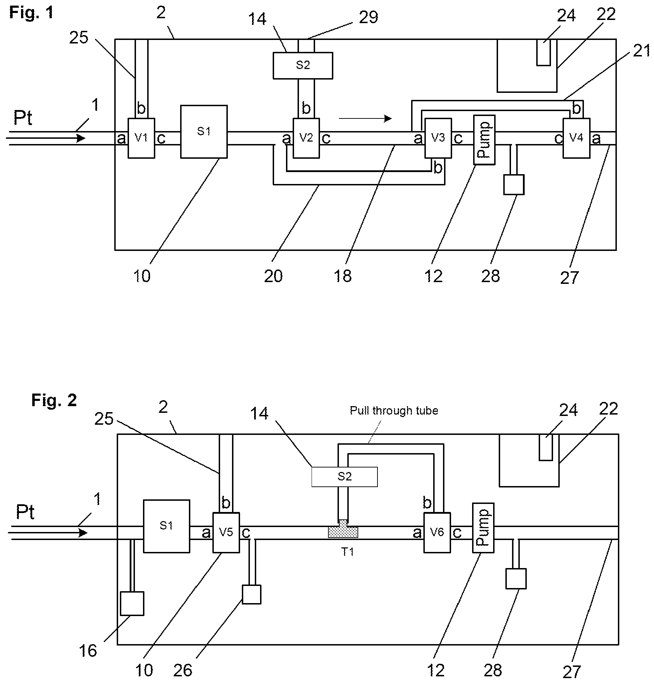

Figure 1 describes schematically an overview of one variation of a device for capturing exhaled breath, including a sampling cannula 1 and a gas sample collection and analysis instrument 2. Gas may be drawn from the patient, for example using a sampling cannula 1 and a flow generator 12. The flow rate of the flow generator may be measured by a flow transducer, for example a pressure sensor array, 26 and 28, arranged like a pneumotach. The measured flow rate may be used as a closed loop feedback control to control the flow generator flow rate. A breath sensor, such as a Capnometer 10 or a pressure sensor 26, is used to measure the breathing pattern in real time. Gas from the desired portion of the breath is captured and isolated in the storage collection compartment 18. Gas entering the storage compartment is controlled by at least one valve V1, for example with a common port c always open, and a second open port, either a to collect gas or b to isolate the storage compartment. There may be a valve V2 between V1 and the flow generator to participate with V1 in isolating the storage compartment. Gas not being captured for analysis is channeled away from the storage compartment via a bypass conduit 20. The captured gas is sent from the storage compartment through a gas composition analyzer 14, such as a NO sensor. A control system 22 with a microprocessor 24 controls the system with the associated algorithms. The flow generator for example can be a vacuum or pressure pump, such as a diaphragm pump, or another type of flow generating device such as a vacuum source, a Venturi from a positive pressure source, or a syringe pump. Valves to manage gas routing can be an arrangement of 3 way 2 position valves as shown, or can be an arrangement of 4-way 3-position valves. Capnometer 10, if used, measures the breathing pattern instantaneously using infrared (IR). The gas composition analyzer for example can be an electrochemical sensor with a reaction time, or a gas chromatographer, a mass spectrometer, a chemiluminescence sensor, an amperometric sensor, or any type of chemical sensor in which the chemistry has been tuned to react to presence of NO. The sensor may measure NO directly, or convert the NO to another molecule such as NO₂ and measure it. The sample storage compartment can be a small bore inner diameter tube or conduit of considerable length in order to minimize the cross section which reduces gas molecule interaction along the length of the conduit. The sampling cannula may be constructed of non-rigid kink-resistant plastic, such as a thermoset plastic for example silicone, urethane or urethane blends, or such as a thermoplastic for example PVC, C-FLEX, or other materials. The cannula can have a range of inner diameters, but preferably less than 080" in order for the breath gas to conform to columnar behavior with a discrete well-defined boundary between breath sections where mixing across may be controlled.

Pressure sensor 16 is an additional pressure sensor that may be used in tandem with 26 so that a flow rate can be determined, in addition to using it for airway pressure measurement. Flow rate can be used to adjust the pump speed in some variations that utilize a variable flow rate. Pressure sensor 16 can also be utilized for ambient information where the breathing curve is measured by pressure instead of Capnometer. In some variations, an instantaneous nitric oxide sensor may be used as the breath sensor, in place of a capnometer or an airway pressure sensor. Other instantaneous breath sensors may also be used.

The bypass tube 20 allows the gas being drawn from the patient or from ambient to bypass the sample tube 18 during times which the sample tube may be isolated from these gases. In this arrangement, valve V1 may be closed at port a and valve V2 may be open at port b to allow flow from b through c. A flow generator may be used to draw the sampling gas through the bypass type. A push tube 21 may be used to push the end-tidal sample in the sample tube 18 out of the sample tube to the sensor 14, at which time valves V1 and V3 are each open at port b and V2 is closed at port a. Valve V4 switches the source gas from patient gas to ambient gas by opening port b, when it is desired to not contaminate the internal gas pathways with patient gas or for purging the system.

In some variations, the pneumatic system shown in Figure 1 above may include a removable sampling compartment (for example, as described in Capnia's provisional patent application 61/872,514, the content of which are incorporated by reference herein in its entirety). For example, sample tube 18 may be removable form the system. In this way, the pneumatic system may be able to fill a sample tube with a desired gas, and the sample tube may be analyzed at another location, or preserved for later analysis. In other variations, the gas may be routed from the sample tube to a removable sampling compartment. In this variation, the compartment may replace the analyzer or otherwise be positioned so that it can be removed and/or replaced.

The control system 22 may include a module or algorithm for performing the breath monitoring and detection function. In this module, a determination is made if the breathing pattern or individual breaths meet certain criteria, in order to determine whether or not a breath will be captured for analysis. The criteria may be predefined, or defined in real-time, or user-defined, automatically defined or semi-automatically defined. For example, predefined criteria may be absolute or relative threshold parameters stored in the device's software. Or a user may enter certain information relative to the specific test being performed, and the system may use that information to define the criteria. Or the system can automatically establish the criteria in real time based on the prevailing conditions. Or a combination of the above techniques can be employed. A subsequent control system module or algorithm within the control system performs the breath sample capturing function, and another subsequent control system module or algorithm performs the breath sample analysis.

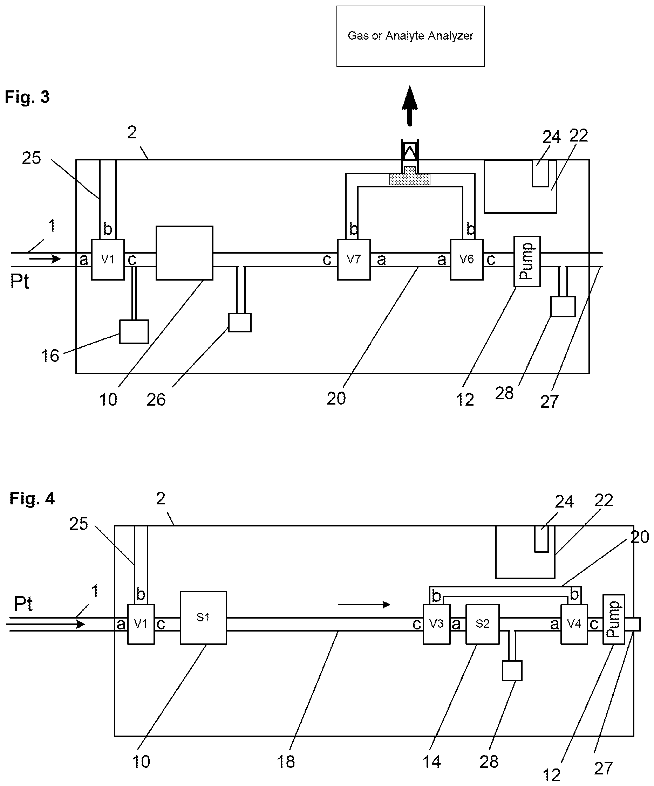

Figure 2 describes an alternative instrument configuration in which exhaled gas is drawn into the instrument by a pump 12 through valves V5 and V6 until a breath is selected for analysis and until the beginning of an appropriate portion of exhaled gas from that selected breath reaches the tee T1. At that time, valve V6 switches from port a to port b and the flow of gas consisting of the appropriate portion of exhaled gas is then routed to sensor S6. When the end of the appropriate portion of exhaled gas reaches the tee T1 valve V6 switches from port b back to port a and the sensor S2 completes its measurement of the concentration of the analyte in the gas sample. Alternatively another valve can be placed before or after tee T1 for additional control and to prevent vacuum and pressure effects, or instead of the tee such as described in Figure 4. In Figure 4 ports b of V3 and V4 are open when not measuring the designated sample and ports a are open when measuring the designated sample.

Figure 3 describes an alternative instrument configuration in which the gas compositional analysis is performed off-board. When the desired section of gas from the desired breath reaches valve V7, V7 and V6 are switched from ports a to ports b, allowing the sample designated for analysis to travel to sample conduit 29. Conduit 29 may be removable so that it can be coupled to the remote analyzer S3, or the instrument 2 may simply connect to the independent analyzer S3. When the designated sample has cleared valve V7, V7 and V6 switch back to ports a so that the measurement of the designated sample is not contaminated with other gases.

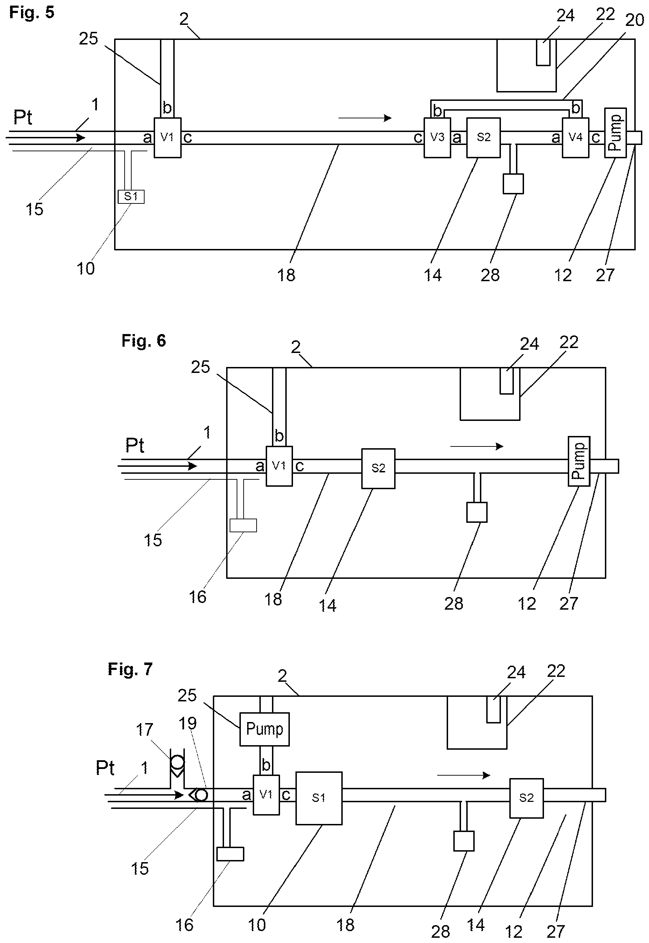

Figure 5 describes an alternative instrument configuration similar to Figure 4 in which there is an additional channel 15 in the patient interface 1, in this case a nasal cannula interface. The sensor S1 is coupled to channel 15 to measure the breathing signal. S1 may be a pressure sensor, flow sensor or other type of sensor. Channel 15 is shown to be not coupled to an active flow source in which case the breathing signal is passively generated by the patient's breathing, however also contemplated the channel 15 may be coupled to a vacuum source to actively draw the gas from the sensor, in which case S1 may be a Capnometer or gas composition sensor. The vacuum source may be the same as pump 12 shown, or another component (not shown) independent of pump 12.

Figure 6 describes an alternative instrument configuration in which a sensor S2 may be used to measure the breathing signal and also used to measure the concentration of the analyte in question. In this case the sensor S2 is a relatively fast responding sensor, such as within 100 msec response time when responding to the analyte in question. This system may still include pressure sensors 16 and 28 to help regulate sampling flow rate, and or as a redundancy to the breathing signal measurement.

Figure 7 shows an alternative instrument configuration in which the collection of exhaled gas is passive, not requiring an active flow source for the collection of the sample. A valve configuration is placed near the patient interface connection to the instrument. The configuration includes an inspiratory valve 17 and an expiratory valve 19. The patient breathes spontaneously through the patient interface, drawing ambient inspired air through valve 17, and exhaling into the instrument through valve 19. The exhaled gas breathing signal is measured by sensor S1 which will identify the different portions of the expiratory phase. When gas from the desired portion of exhalation passes through sensor S2 it is measured for its concentration of the analyte. A pump 25 is provided to purge the system with ambient air, or to measure the gas in question in the ambient air for correction. Of course the configuration in Figure 7 can be combined with other alternative instrument configurations for example Figure 5 in which there is a bypass 20 around the sensor S2 for all other gases other than the designated sample.

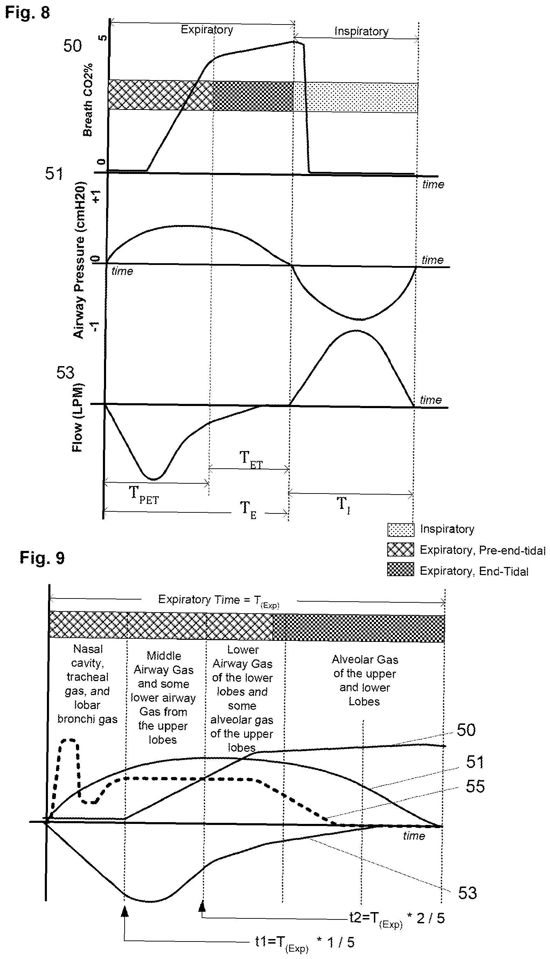

Now referring to Figure 8, typical breathing signals are shown graphically as a function of time for an expiratory phase of one breath and the inspiratory phase of the subsequent breath. The top, middle and lower tracing correspond to a Capnometer signal, airway pressure signal and flow signal respectively, for a normal tidal volume breath. The CO₂ signal can be from a side-stream sensor such as S1 in Figure 5, or a mainstream CO₂ sensor, and the airway and flow sensors can be at the patient airway or a distance away from the airway. During the expiratory phase E, CO₂ is expelled, hence the CO₂ level increases. During the inspiratory phase I, ambient air occupies the upper airways, hence the measured CO₂ drops to essentially zero. There may be a variety of shapes to a breath CO₂ curve, based on the person's breathing pattern, their age, how they are breathing and any underlying acute or chronic medical conditions. A classic curve may show the following sub-portions for the expiratory phase: (1) a beginning portion or pre-end-tidal section PET, comprising low or little CO₂ because the gas may simply be gas from the proximal airway devoid of CO₂, (2) a middle portion showing CO₂ rapidly increasing from zero to the CO₂ level at the distal segments of the lungs, and (3) an end-tidal ET portion showing a plateauing or leveling off of the CO₂, representing the CO₂ coming from the alveoli for that exhaled breath, and (4) potentially a constant peak level at the very end of the expiratory period. However, there can be many other curves different from this classic curve. Peak CO₂ levels are typically 4-6% during the end-tidal period and close to or equal to zero during the inspiratory period.

In some variations, the level of CO₂ in an exhaled breath may be used to determine the duration of a period of a breath, such as the pre-end-tidal time TPET, expiratory time TE, end-tidal time TET, inspiratory time TI, or breath period time TBP. In further variations, a duration of a period of breath may be characterized by a start and a termination of that period. In some variations, a CO₂ level may be used to determine a start or a termination of a period of a breath. In other variations, a first time derivative of a CO₂ level may be used to determine a start or a termination of a period of a breath. In yet other variations, a second time derivative of a CO₂ level may be used to determine a start or a termination of a period of a breath. In some variations, a combination of CO₂ levels and CO₂ level time derivatives may be used to determine a start or a termination of a period of a breath. In some variations, a start of an end-tidal period may be determined by a change in the first time derivative of a CO₂ level of the exhaled breath, such as a sudden decrease in the first time derivative of the CO₂ level. In some variations, a decrease in the first time derivate of the CO₂ level may be more than a 10% decrease. In some variations, a decrease in the first time derivate of the CO₂ level may be more than a 25% decrease. In some variations, the derivative will approach or become zero showing very little rate of change or a peak plateau respectively. In other variations, the start of an end-tidal period may be determined by a large second time derivative of the CO₂ level. In some variations, a termination of an end-tidal period may be determined by a maximum CO₂ level, which may be detected or confirmed by a change in the sign of the first time derivative of the CO₂ level as the derivative becomes negative associated with a drop of the CO₂ level from its peak value. In further variations, a start of a beginning period may be determined by a sudden increase in the first time derivative of the CO₂ level. In other variations, the start of a beginning period may be determined by an increase in the CO₂ level from zero CO₂ level. In some variations, a termination of a middle period may be determined by a change in the first time derivative of a CO₂ level of the exhaled breath, such as a sudden decrease in the first time derivative of the CO₂ level. In some variations, a CO₂ level, first time derivative thereof, or second time derivative thereof may be used to determine the start and termination of one or more periods. Other breath-borne gases may be used in place of CO₂ for measuring the breathing curve. For example, oxygen can be measured which would indicate a higher oxygen concentration during inspiration than expiration. In some variations, the breathing pattern may be instantaneously or substantially instantaneously measured by a fast-responding NO sensor. In this case referring to Figure 1, the sensor 10 may be a fast responding NO sensor that depicts the breathing pattern and also measures the end-tidal NO level. After application of the various breath qualification and disqualification variations described subsequently, the NO level of a qualified breath can be reported as the result.

In the middle tracing of Figure 8, the measured airway pressure signal may be from sensor 10 in Figure 5, showing a negative pressure during inspiratory phase and a positive pressure during expiratory phase. Typically during at rest breathing the peak expiratory pressure may correspond to the middle of the expiratory phase and the start of the end-tidal period. In Figure 8, TI, TE, TPET, TET, TPE represent inspiratory time, expiratory time, pre-end-tidal time, end-tidal time, and post expiratory time respectively. An inspiratory pause may also be present (not shown), in which the peak of lung muscle movement during inspiration is paused before the expiratory period begins. Peak inspiratory pressure may be - 1 to -4 cwp during restful breathing, and up to -15 cwp during heavier breathing, and peak expiratory pressure may be +0.5 to +2.0 cwp during restful breathing and up to +10 cwp during heavier breathing when measured at the entrance to the nostrils. Representative pressures and gas concentrations may vary with environmental conditions, for example airway pressures during cold temperatures may be increased for the same unit of volume. In the lower tracing of Figure 8 a breathing flow rate is measured for example from Sensor S1 in Figure 5.

In some variations, airway pressure may be used to determine a start or a termination of a period of a breath. In other variations, a first time derivative of an airway pressure may be used to determine a start or a termination of a period of a breath. In yet other variations, a second time derivative of an airway pressure may be used to determine a start or a termination of a period of a breath. In some variations, a combination of airway pressures and airway pressure time derivatives may be used to determine a start or a termination of a period of a breath. In some variations, a start of an end-tidal period may be determined by maximum airway pressure, that is, by a zero first time derivative of the airway pressure. In some variations, a termination of an end-tidal period may be determined by zero airway pressure. In some variations, an airway pressure, first time derivative thereof, or second time derivative thereof may be used to determine the start and termination of one or more periods. Airway pressure may be measured through a secondary lumen extending the length of the cannula in parallel with the sampling lumen, or may be measured by teeing into the sampling lumen, or by placing a sensing transducer at the airway of the patient.

In some variations, the breath sensor monitors the person's breathing over time, and trends the breathing pattern by determining a continually updated value that is characteristic of the breathing pattern. For example, peak positive values of a breathing signal may be measured and updated for each breath. Peak values may be compared with previous peak values. Peak values may be averaged over a previous number of multiple breaths. Similarly, time-related aspects of the breaths may be trended, such as the expiratory time. Various breath-related events that are not normal breaths may be identified and exception algorithms may exist in order to not include these non-normal breath events inadvertently in deterministic steps. For example, the characteristic waveform of a sneeze, cough, stacked breath, or non-full breath may be defined in advance or based on monitoring of a particular patient, and when detected by the breathing sensor, discarded by the appropriate deterministic algorithms.

Now referring to Figure 9 the expiratory phase of breathing is graphically partitioned into physiologically different partitions, corresponding to different anatomical regions of the pulmonary tree of the lung. A Capnometer tracing 50, airway pressure tracing 51 and flow rate tracing 53 are shown, as well as a theoretical NO concentration tracing 55 of a patient with an exacerbation of airway inflammation indicative of an ensuing asthma attack. The scale of the NO tracing can typically be from 0 to 300 ppb, although higher levels may be measured as well. In the example shown, 5 partitions are created for a single expiration, in this case a normal tidal volume breath such as that shown in Figure 8. While 5 partitions are shown, there may be less or more partitions depending on the clinical test being undertaken and the prevailing conditions surrounding the test. In the example shown, the first partition is the section of exhalation corresponding to oro-nasal volume, tracheal volume, and lobar bronchi volume. The second partition is the section of exhalation corresponding to lung airways in the middle of the lung's lower lobes and lower part of the lung's upper lobes. For example, these are the airways between the segmental bronchi to the 6^{th} branching generation of the pulmonary tree, inclusive. These airways in particular may be prone to the inflammation that occurs in asthma. These airways may also be referred to the conducting airways, being the conduits responsible for conducting the respiratory gases to and from the gas exchange areas in the distal most regions of the lung, known as the alveoli. The third of five partitions is the section of exhaled gas corresponding to the lower airways of the lower lobes of the lung and some alveolar gas from the upper lobes. The fourth and fifth partitions contain increasing percentages of alveolar gas until the gas is 100% pure alveolar gas. Because of the non-uniform architecture of the lung's branching structure, gas from different lobes reaches the conducting airways at different times and therefore, the partitioning of the expiratory gas into different sections of the bronchopulmonary tree includes some heterogeneous zones where alveolar gas and conducting airway gas is being expelled out of the nose or mouth at the same time. Therefore, this heterogeneity may be taken into account when partitioning the expiratory phase into different anatomical or physiological sections. For example as shown in Figure 8, in the third partition contains both airway gas and some alveolar gas hence it may be beneficial to limit analyzing the gas from this section. Further, the gas from the second partition, based on this graphical example, appears to be a good section from which to collect NO gas for analysis. In some variations, the inflammatory process involves the lower airways of the lung, and these variations may be used to identify, partition and analyze gas from those lower airways even if that gas, when exiting the patient's nose or mouth, may contain some alveolar gas from the upper lobes.

Still referring to Figure 9, the sensor used to measure the breathing signal is used to assign timestamp values to the start and end of each partition of the expiratory phase. The timestamp values will allow the system to know the location of each partition inside the instrument at any given time as explained elsewhere. There are two basic approaches contemplated to assign time stamp values to each partition. In a first approach, the expiratory duration is determined and after the expiratory phase is complete, this duration is divided into the required number of sections. The duration of the expiratory phase can be determined after gas from an expiratory phase is drawn through the sensor S1, by marking the beginning and end of the expiratory phase, t(i) and t(f) respectively and setting the duration t(exp) = t(f) - t(i). If the expiratory phase is to be divided into five partitions as in this example, section 1 will be identified and located as timestamp values t(i) through [ t(i) + t(exp)/5], section 2 will be [t(i) + t(exp)/5] through [t(i) + t(exp)^{∗}4/5], and so on. In a second approach for assigning timestamp values, the beginning and end of each expiratory partition is determined in real time as the expiratory gas travels through or to the sensor S1 or 16. The timestamps of the different partitions are determined by characteristics in the breath sensor signal or the processed signal, such as a sudden increase or decrease, a crossing of zero, a slope requirement, or other characteristics. One or more sensors can be used to obtain enough information to make the timestamp assignments, and the signals can be amplified, differentiated, transformed or converted in other ways as explained elsewhere. A pressure sensor can be used to determine the start and end of the expiratory phase, by a positive slope crossing zero and a negative slope crossing zero respectively. Or the drop in a CO₂ signal can demark the end of an expiratory cycle. Inflection points of the airway pressure or CO₂ signals may mark intermediate points along the expiratory phase. In the example shown the beginning and end of the second partition is given time stamp values of t1 and t2 respectively. For example t(1) may be determined by an increase in the CO₂ signal, and t(2) can be determined by a peak in the airway pressure signal. In addition to using the first or second approach to time-stamping, the two approaches can be combined. These time stamps will allow the instrument to know the location of the gas from that section of breath while inside the instrument at any given time.

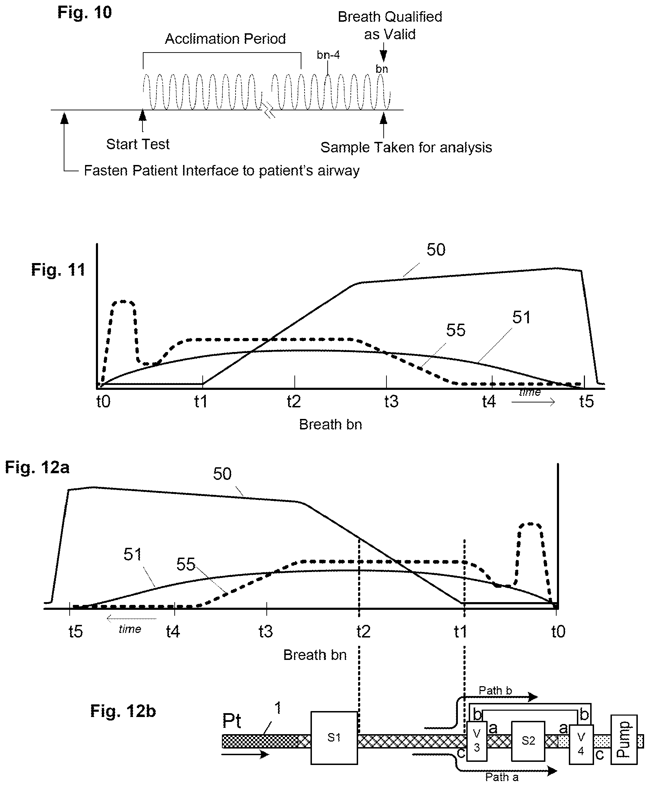

In Figure 10 an example of an overall test sequence is graphically described as a function of time. Before starting the test the patient interface such as a nasal cannula is fastened to the patient and coupled to the airway. After the test is started, the patient is instructed to breathe normally and a breathing sensor monitors the breathing pattern to verify stable breathing. The breathing pattern is shown by the tracing and can be for example Capnometer, airway pressure, flow or other breathing parameters. The instrument verifies that the breathing is stable according to a set of criteria. The criteria will insure steady state gas diffusion conditions in the lung exist so that a physiologically accurate NO sample can be obtained. After the appropriate acclimation period criteria are met, the instrument begins to look for a valid breath for sampling for the NO measurement. In order to be classified as a valid breath, the breath characteristics may be required to meet a set of criteria to ensure that the sample is taken from a physiologically representative breath. Breaths classified as not valid will be rejected for analysis. In the example shown, breath bn is determined as valid and is targeted for sample acquisition. The instrument's partitioning algorithms then isolate the desired section of exhaled gas from breath bn for compositional analysis, for example the middle airway gas for NO analysis.

Figure 11 shows the exhalation phase of breath bn in Figure 10 in more detail, showing the Capnometer, airway pressure, flow and theoretical NO levels 50, 51, 53 and 55 respectively versus time. In Figure 12a, the graph shown in Figure 11 is shown with the timescale flipped, such that the most recent time point of the expiratory tracing of breath bn is on the left hand side of the graph. Figure 12b is a pneumatic schematic of the instrument shown in Figure 5, in which the sample tube 18 is schematically aligned with the graph shown in Figure 12a. In Figures 10 through 12b, the second fifth of the expiratory cycle is the portion of the expiratory cycle that is targeted in breath bn for NO measurement, to represent the NO level in the middle airways. As can be seen, the sample of gas between time points t1 and t2 is drawn into the sample tube 18 between sensor S1 and valve V3. Before gas from time point t1 reaches valve V3 the gas flow path is path b around the sensor S2 and out the pump, however, once gas from time point t1 reaches valve V3, the control system changes the valve positions to ports a and the gas flow path is then path a so that the middle airway gas sample from the second exhalation zone travels through sensor S2 for compositional analysis. Once the gas from time point t2 reaches the valve V3, the control systems changes the valve positions again to port b so that the remaining gas bypasses the sensor S2 by flowing through path b, so that this gas does not interfere with the middle airway NO measurement. As explained previously, because the pump flow rate, system volumes, and time between t1 and t2 are known, the control system is able to switch the valve porting at the correct times. Typically, there is a delay between the time at which gas from time point t1 exits the sensor S1 and the time it reaches valve V3 for example 100 msec. The timestamps t(1) and t(2) are determined in real time or near real time as the gas travels through the sensor S1 and before the gas reaches valve V3. Before the second section reaches valve V3 the gas travels in path b around the sensor S2 and when the second section reaches valve V3 the valves are switched to port a and the gas travels through the sensor S2 in path a. When the end of the second section of gas clears valve V3, the valves are switched back to port b and the subsequent gas travels around the sensor S2 to limit contaminating the measurement of the second section of gas. The distance between valve V3 and sensor S2 may be taken into account in the valve control timing so that only the desired gas reaches and is measured by sensor S2.

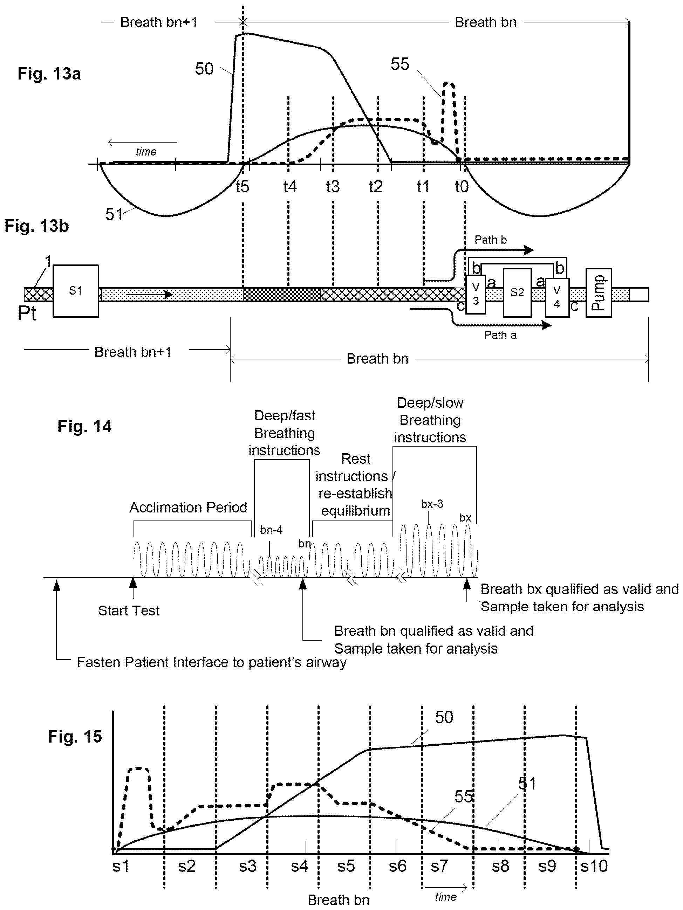

As explained previously, the timestamp values can be determined and assigned in real time as the expiratory gas is traveling through sensor S1, or can be done after all the exhaled gas has traveled through the sensor S1 so that the system has a chance to measure the complete expiratory cycle, or a combination of the above. In Figure 13a and 13b an example of analyzing the second expiratory partition from breath bn from Figure 10 is shown, and in which the entire expiratory cycle of breath bn is drawn past the sensor S1 into the sample tube 18 prior to reaching the valve V3. The timestamp values required to identify the start and stop of the second expiratory partition are determined for this section of gas before it reaches valve V3, using the breathing sensor signal and techniques described elsewhere. Before the second section reaches valve V3 the gas travels in path b around the sensor S2 and when the second section reaches valve V3 the valves are switched to port a and the gas travels through the sensor S2 in path a. When the end of the second section of gas clears valve V3, the valves are switched back to port b and the subsequent gas travels around the sensor S2 to limit contaminating the measurement of the second section of gas. The distance between valve V3 and sensor S2 may be taken into account in the valve control timing so that only the desired gas reaches and is measured by sensor S2.

Figure 14 describes another variation in which gas samples are taken from more than one type of breathing pattern, and analyzed and compared. The comparison may increase the sensitivity and specificity of the test for a specific diagnosis. Optionally, samples can be collected for more than two types of breathing patterns, for example fast, slow and normal. Typically there are acclimation periods before collecting the samples so that steady state conditions are obtained and typically the instrument instructs the patient on how to breathe and verifies the patient is breathing as required to validate a test sequence.

It is contemplated that different levels of lung airways in asthma may be prone to the inflammatory response arising from a specific type of asthma or a specific irritant or exacerbation. For example inflammation of the segmental bronchi may correlate to a certain type of asthma, or a certain type of irritant causing an attack. And similarly, inflammation of the lower airways, such as the 6^{th} - 8^{th} generation of the airway branching structure, may correlate to a different type of asthma or a different irritant. Therefore, some variations may be useful in finding which portion of the bronchopulmonary tree is most affected by the inflammation. This information can help the clinician determine the optimal treatment and even a cure. For example, for bronchoplasty treatment, the measurements obtained from the systems and methods herein can help inform the interventional pulmonologist on which airways in the lung may need to be treated, and can therefore optimize treatment, stage treatments over time, and avoid over-treating or undertreating. Figure 15 graphically describes a hypothetical gradient of NO gas in the expiratory phase. In this example the expiratory phase is divided into 10 sections s1 through s10 in order to partition the expiratory phase into enough sections to determine an airway NO gradient. In section s4 there is a peak in airway NO (aNO) of 50ppb, indicating the most inflammation is found to be associated with this section which may represent for example the subsegmental airways, and which is indicative of a certain hypothetical genotype of asthma or a form of asthma prone to a certain irritant. In this case the instrument shown in Figure 5 is adapted to take aNO measurements from all the sections s1 through s10 either from one breath or from multiple breaths including each section from one dedicated breath.

In another variation, the contribution of nasal NO to conducting airways NO may be taken into account. For example, referring to Figure 15, the nasal NO may be measured at 100ppb in a first test measuring the nasal NO using the techniques described herein. Then, in a second test of a similar breath, or in the same test as the nasal NO test, the NO in the conducting airways may be measured at 10ppb, however, it may contain some nasal NO. After an inspiration, assuming for example the conducting airways contains for example 2% nasal gas drawn into the airways during inspiration and 98% ambient air, based on breath rate information and possibly other factors, then during exhalation the conducting airways NO can be determined by the equation: NO_{(CAtotal)} = 2%NO(_{CAnasal}) + 98%NO_{(CA)}, where NO_{(CAtotal)}, NO_{(CAnasal)} and NO_{(CA)} equals NO measured in the conducting airways, NO measured in the nasal sinuses and NO arising from the conducting airways, respectively. Therefore, 10ppb_{(CAtotal)} = 0.02x100ppb_{(CAnasal)} + 0.98xNO_{(CA)} , or NO_{(CA)} = 8.89ppb.

In another variation, ambient NO may be measured during gas collected via the cannula during the inspiratory phase of breathing, and the conducting airways NO measurement may be corrected for the ambient NO measurement.

In another variation, the techniques described herein may provide different measured aNO values compared to the current clinical practice FENO values, even within intra-patient values. Therefore, a correlation of aNO values obtained with this techniqueto FENO values obtained with conventional FENO values is first established. This can be done by performing adult measurements, pediatric measurements, as well as measurements on younger pediatric and infant patients who cannot follow the breathing instructions of the conventional techniques. For the latter, the measurements can be collected by trial and error over the course of a number of attempted measurements for a given test subject, and a valid measurement obtained. After the correlation of aNO to FENO is established, the apparatuses described herein may be able to report to the user both the measured aNO value just measured, and, based on the previously established correlations, report the estimated FENO value for comparison.

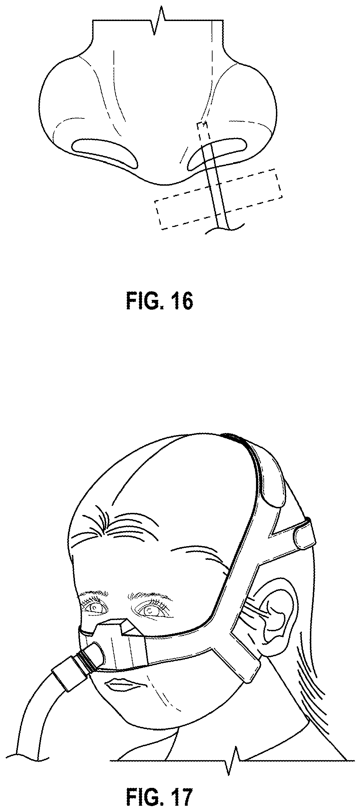

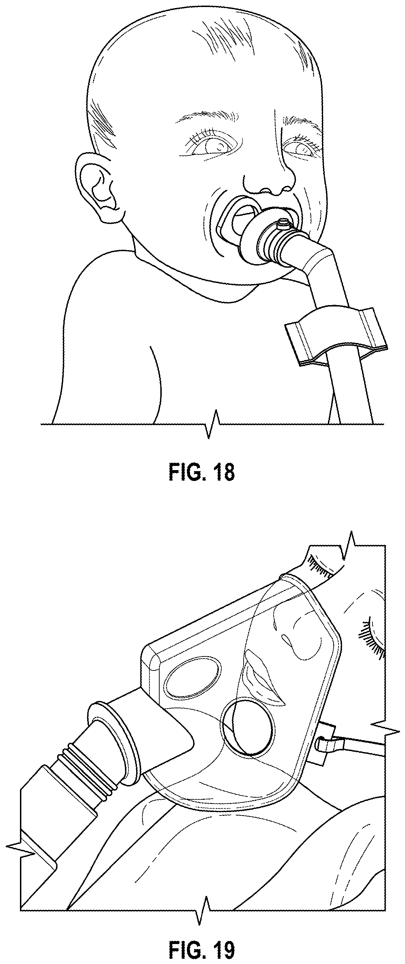

Figures 16-19 describe different patient interfaces that may be used to collect the exhaled gas sample from the patient including a nasal cannula, nasal mask, oral mask, and facemask.

In the foregoing descriptions of variations, it should be noted that it is also conceived that the sequences of operation described in the Figures can be combined in all possible permutations. In addition, while the examples describe aNO measurements they may apply to other gases and analytes. The examples provided throughout are illustrative of the principles of the systems and methods described herein, and that various modifications, alterations, and combinations can be made by those skilled in the art without departing from the scope and spirit of the invention. Any of the variations of the various breath measurement and sampling devices disclosed herein can include features described by any other breath measurement and sampling devices or combination of breath measurement and sampling devices herein. Accordingly, it is not intended that the invention be limited, except as by the appended claims. For all of the variations described above, the steps of the methods need not be performed sequentially.

## Claims

1. An apparatus for analyzing nitric oxide levels in breath, comprising:
a nasal sampling cannula (1);
a breathing sensor to measure a breathing pattern parameter;
a breath sampling system comprising a plurality of valves (V1-V7) to isolate sections of the breath gas sampling system;
a pump (12) downstream from at least two of the plurality of valves (V1-V7) that draws a plurality of breaths from a patient into the nasal sampling cannula (1) and through the breath sampling system;
a first processor containing executable instructions for analyzing breathing pattern parameters of the plurality of breaths;
a second processor containing executable instructions for establishing in real-time, based on the analyzed breathing pattern parameters of the plurality of breaths, a breathing pattern parameter criteria that delineates between a physiologically representative breath and a physiologically non-representative breath;
a third processor containing executable instructions for determining whether a breathing pattern parameter of a breath, exhaled after the plurality of breaths, meets the breathing pattern parameter criteria;
a fourth processor containing executable instructions for, in response to a determination that a breathing pattern parameter of the breath meets the breathing pattern parameter criteria, delineating at least a first section of the breath, a second section of the breath, and a third section of the breath, wherein the first section of breath is exhaled before the second section of breath and the second section of breath is exhaled before the third section of breath;
a fifth processor containing executable instructions for controlling the plurality of valves (V1-V7) to isolate the first section of breath, second section of breath, and third section of breath from each other; and
an analyzer (14) to analyze nitric oxide levels in at least one of the second section of the breath and the third section of the breath separately from the other.

2. The apparatus of Claim 1, wherein the executable instructions for controlling the plurality of valves comprise executable instructions for discarding the first section of breath.

3. The apparatus of Claim 1, wherein the first, second, third, fourth, and fifth processors comprise a single processor.

4. The apparatus of Claim 1, wherein the breathing pattern parameter criteria comprise a measurement of a breath rate stability parameter as a function of time and a measurement of a respiratory frequency parameter.

5. The apparatus of Claim 1, wherein the second section of breath corresponds to a section of the bronchopulmonary tree between a proximal section and a distal section, or wherein the second section of breath corresponds to a section of the respiratory tract tree selected from the group consisting of: the nasal airway, the trachea, the main stem bronchii, the segmental bronchii, the conducting airways, the respiratory airways, and the alveoli.

6. The apparatus of Claim 5, wherein the proximal section is a main stem bronchus and the distal section is one selected from group consisting of a 4th to 8th branching structure.

7. The apparatus of Claim 1, wherein the executable instructions for delineating at least a first section of the breath, a second section of the breath, and a third section of the breath comprise executable instructions for delineating a fourth section of the breath and a fifth section of the breath, or comprise executable instructions for determining a duration of at least a part of an exhalation phase and dividing the duration into time sections corresponding to the first section of the breath, the second section of the breath, and the third section of the breath, or comprise executable instructions for determining characteristics in the breathing sensor signal, the characteristics selected from the group consisting of: a zero signal amplitude, a peak signal amplitude, a crossing of zero from a negative value to a positive value, a crossing of zero from a positive value to a negative value, a plateau in the signal amplitude, a change in slope of the signal amplitude, a zero of the differential of the signal, a peak of the differential of the signal, a zero of the second differential of the signal, a peak of the second differential of the signal, a zero of a transform of the signal, a peak of a transform of the signal.

8. The apparatus of Claim 1, further comprising:
a) a sixth processor comprising executable instructions for notifying a patient to breathe at a desired frequency; and a seventh processor comprising executable instructions for verifying that the patient is breathing at the desired frequency, wherein the executable instructions for determining whether a breathing pattern parameter of the breath meets the breathing pattern parameter criteria comprise executable instructions for collecting the breath following a verification that the patent is breathing at a desired frequency;
b) an eighth processor comprising executable instructions for measuring the nitric oxide in at least two sections of the breath and correcting the level of nitric oxide measured in a physiologically valid section with the nitric oxide measured in another section; or
c) a ninth processor comprising executable instructions for measuring the analyte in multiple breaths for determining a final compositional value.

9. A method of analyzing nitric oxide levels in breath, comprising:
drawing a plurality of breaths from a patient breathing normally using a nasal sampling cannula (1), where a pump (12) is used to draw the plurality of breaths;
analyzing breathing pattern parameters of the plurality of breaths;
establishing in real-time, based on the analyzed breathing pattern parameters of the plurality of breaths, a breathing pattern parameter criteria that delineates between a physiologically representative breath and a physiologically non-representative breath;
determining whether a breathing pattern parameter of a breath, exhaled after the plurality of breaths, meets the breathing pattern parameter criteria;
in response to a determination that a breathing pattern parameter of the breath meets the breathing pattern parameter criteria, delineating at least a first section of the breath, a second section of the breath, and a third section of the breath, wherein the first section of breath is exhaled before the second section of breath and the second section of breath is exhaled before the third section of breath;
controlling a plurality of valves (V1-V7) to isolate the first section of breath, second section of breath, and third section of breath from each other; and
analyzing nitric oxide levels in at least one of the second section of the breath and the third section of the breath separately from the other.

10. The method of Claim 9, wherein controlling the plurality of valves comprises discarding the first section of breath.

11. The method of Claim 9, wherein the breathing pattern parameter criteria comprise a measurement of a breath rate stability parameter as a function of time and a measurement of a respiratory frequency parameter.

12. The method of Claim 9, wherein the second section of breath corresponds to a section of the bronchopulmonary tree between a proximal section and a distal section, or wherein the second section of breath corresponds to a section of the respiratory tract tree selected from the group consisting of: the nasal airway, the trachea, the main stem bronchii, the segmental bronchii, the conducting airways, the respiratory airways, and the alveoli.

13. The method of Claim 12, wherein the proximal section is a main stem bronchus and the distal section is one selected from group consisting of a 4th to 8th branching structure.

14. The method of Claim 9, wherein delineating at least a first section of the breath, a second section of the breath, and a third section of the breath comprises:
delineating a fourth section of the breath and a fifth section of the breath, or
determining a duration of at least a part of an exhalation phase and dividing the duration into time sections corresponding to the first section of the breath, the second section of the breath, and the third section of the breath, or
determining characteristics in the breathing sensor signal, the characteristics selected from the group consisting of: a zero signal amplitude, a peak signal amplitude, a crossing of zero from a negative value to a positive value, a crossing of zero from a positive value to a negative value, a plateau in the signal amplitude, a change in slope of the signal amplitude, a zero of the differential of the signal, a peak of the differential of the signal, a zero of the second differential of the signal, a peak of the second differential of the signal, a zero of a transform of the signal, a peak of a transform of the signal.

15. The method of Claim 11, further comprising:
a) notifying a patient to breathe at a desired frequency; and
verifying that the patient is breathing at the desired frequency, wherein determining whether a breathing pattern parameter of the breath meets the breathing pattern parameter criteria comprises collecting the breath after a verification that the patient is breathing at a desired frequency;.or
b) measuring the nitric oxide in at least two sections of the breath; and
correcting the level of nitric oxide measured in a physiologically valid section with the nitric oxide measured in another section; or
c) measuring the analyte in multiple breaths for determining a final compositional value.

## Patentansprüche

1. Vorrichtung zum Analysieren von Stickoxidkonzentrationen in Atem, umfassend:
eine nasale Probenahmekanüle (1);
einen Atemsensor zum Messen eines Atemmusterparameters;
ein Atemprobenahmesystem, das eine Mehrzahl von Ventilen (V1-V7) zum Trennen von Teilen des Atemgasprobenahmesystems umfasst;
eine Pumpe (12) stromabwärts von mindestens zweien der Mehrzahl von Ventilen (V1-V7), die eine Mehrzahl von Atemzügen von einem Patienten in die nasale Probenahmekanüle (1) und durch das Atemprobenahmesystem ansaugt;
einen ersten Prozessor, der ausführbare Anweisungen zum Analysieren von Atemmusterparametern der Mehrzahl von Atemzügen enthält;
einen zweiten Prozessor, der ausführbare Anweisungen zum Festlegen eines Atemmusterparameterkriteriums, das zwischen einem physiologisch repräsentativen Atem und einem physiologisch nicht repräsentativen Atem abgrenzt, basierend auf den analysierten Atemmusterparametern der Mehrzahl von Atemzügen in Echtzeit enthält;
einen dritten Prozessor, der ausführbare Anweisungen zum Bestimmen enthält, ob ein Atemmusterparameter eines nach der Mehrzahl von Atemzügen ausgeatmeten Atems das Atemmusterparameterkriterium erfüllt;
einen vierten Prozessor, der ausführbare Anweisungen zum Abgrenzen mindestens eines ersten Teils des Atems, eines zweiten Teils des Atems und eines dritten Teils des Atems in Reaktion auf eine Bestimmung enthält, dass ein Atemmusterparameter das Atemmusterparameterkriterium erfüllt, wobei der erste Atemteil vor dem zweiten Atemteil ausgeatmet wird, und der zweite Atemteil vor dem dritten Atemteil ausgeatmet wird;
einen fünften Prozessor, der ausführbare Anweisungen zum Steuern der Mehrzahl von Ventilen (V1-V7) zum Trennen des ersten Atemteils, des zweiten Atemteils und des dritten Atemteils voneinander enthält; und
einen Analysator (14), um Stickoxidkonzentrationen in mindestens einem des zweiten Teils des Atems und des dritten Teils des Atems getrennt voneinander zu analysieren.

2. Vorrichtung nach Anspruch 1, wobei die ausführbaren Anweisungen zum Steuern der Mehrzahl von Ventilen ausführbare Anweisungen zum Verwerfen des ersten Atemteils umfassen.

3. Vorrichtung nach Anspruch 1, wobei der erste, der zweite, der dritte, der vierte und der fünfte Prozessor einen einzigen Prozessor umfassen.

4. Vorrichtung nach Anspruch 1, wobei das Atemmusterparameterkriterium eine Messung eines Atemfrequenzstabilitätsparameters in Abhängigkeit von der Zeit und eine Messung eines Respirationsfrequenzparameters umfasst.

5. Vorrichtung nach Anspruch 1, wobei der zweite Atemteil einem Teil des Bronchopulmonalbaums zwischen einem proximalen Teil und einem distalen Teil entspricht, oder wobei der zweite Atemteil einem Teil des Respirationstraktbaums entspricht, der ausgewählt ist aus der Gruppe bestehend aus: dem Nasenweg, der Luftröhre, den Hauptstammbronchien, den Segmentbronchien, den leitenden Atemwegen, den Respirationswegen und den Luftbläschen.

6. Vorrichtung nach Anspruch 5, wobei der proximale Teil ein Hauptstammbronchus ist, und der distale Teil einer ist, der aus der Gruppe bestehend aus einer 4. bis 8. Verzweigungsstruktur ausgewählt ist.

7. Vorrichtung nach Anspruch 1 wobei die ausführbaren Anweisungen zum Abgrenzen mindestens eines ersten Teils des Atems, eines zweiten Teils des Atems und eines dritten Teils des Atems ausführbare Anweisungen zum Abgrenzen eines vierten Teils des Atems und eines fünften Teil des Atems umfassen oder ausführbare Anweisungen zum Bestimmen einer Dauer mindestens eines Teils einer Ausatmungsphase und Teilen der Dauer in Zeitabschnitte umfassen, die dem ersten Teil des Atems, dem zweiten Teil des Atems und dem dritten Teil des Atems entsprechen, oder ausführbare Anweisungen zum Bestimmen von Charakteristiken im Atemsensorsignal umfassen, wobei die Charakteristiken ausgewählt sind aus der Gruppe bestehend aus: einer Nullsignalamplitude, einer Spitzensignalamplitude, einem Nulldurchgang von einem negativen Wert zu einem positiven Wert, einem Nulldurchgang von einem positiven Wert zu einem negativen Wert, einem Plateau in der Signalamplitude, einer Änderung der Steilheit der Signalamplitude, einem Nullpunkt der Differenz des Signals, einer Spitze der Differenz des Signals, einem Nullpunkt der zweiten Differenz des Signals, einer Spitze der zweiten Differenz des Signals, einem Nullpunkt einer Transformierten des Signals, einer Spitze einer Transformierten des Signals.

8. Vorrichtung nach Anspruch 1, ferner umfassend:
a) einen sechsten Prozessor, der ausführbare Anweisungen zum Benachrichtigen eines Patienten umfasst, dass er mit einer gewünschten Frequenz atmen soll; und einen siebten Prozessor, der ausführbare Anweisungen zum Verifizieren umfasst, dass der Patient mit der gewünschten Frequenz atmet, wobei die ausführbaren Anweisungen zum Bestimmen, ob ein Atemmusterparameter des Atems das Atemmusterparameterkriterium erfüllt, ausführbare Anweisungen zum Sammeln des Atems nach einer Verifizierung umfasst, dass der Patient mit einer gewünschten Frequenz atmet;
b) einen achten Prozessor, der ausführbare Anweisungen zum Messen des Stickoxids in mindestens zwei Teilen des Atems und Korrigieren der Konzentration des in einem physiologisch gültigen Teil gemessenen Stickoxids mit dem in einem anderen Teil gemessenen Stickoxid umfasst; oder
c) einen neunten Prozessor, der ausführbare Anweisungen zum Messen des Analyten in mehreren Atemzügen zum Bestimmen eines zusammengesetzten Endwerts umfasst.

9. Verfahren zum Analysieren von Stickoxidkonzentrationen in Atem, umfassend:
Ansaugen einer Mehrzahl von Atemzügen aus einer Atmung eines Patienten unter normaler Verwendung einer nasalen Probenahmekanüle (1), wobei eine Pumpe (12) zum Ansaugen der Mehrzahl von Atemzügen verwendet wird;
Analysieren von Atemmusterparametern der Mehrzahl von Atemzügen;
Festlegen eines Atemmusterparameterkriteriums, das zwischen einem physiologisch repräsentativen Atem und einem physiologisch nicht repräsentativen Atem abgrenzt, basierend auf den analysierten Atemmusterparametern der Mehrzahl von Atemzügen in Echtzeit;
Bestimmen, ob ein Atemmusterparameter eines nach der Mehrzahl von Atemzügen ausgeatmeten Atems das Atemmusterparameterkriterium erfüllt;
Abgrenzen mindestens eines ersten Teils des Atems, eines zweiten Teils des Atems und eines dritten Teils des Atems in Reaktion auf eine Bestimmung, dass ein Atemmusterparameter das Atemmusterparameterkriterium erfüllt, wobei der erste Atemteil vor dem zweiten Atemteil ausgeatmet wird, und der zweite Atemteil vor dem dritten Atemteil ausgeatmet wird;
Steuern einer Mehrzahl von Ventilen (V1-V7), um den ersten Atemteil, den zweiten Atemteil und den dritten Atemteil voneinander zu trennen; und
Analysieren von Stickoxidkonzentrationen in mindestens einem des zweiten Teils des Atems und des dritten Teils des Atems getrennt voneinander.

10. Verfahren Anspruch 9, wobei das Steuern der Mehrzahl von Ventilen ein Verwerfen des ersten Atemteils umfasst.

11. Verfahren nach Anspruch 9, wobei das Atemmusterparameterkriterium eine Messung eines Atemfrequenzstabilitätsparameters in Abhängigkeit von der Zeit und eine Messung eines Respirationsfrequenzparameters umfasst.

12. Verfahren nach Anspruch 9, wobei der zweite Atemteil einem Teil des Bronchopulmonalbaums zwischen einem proximalen Teil und einem distalen Teil entspricht, oder wobei der zweite Atemteil einem Teil des Respirationstraktbaums entspricht, der ausgewählt wird aus der Gruppe bestehend aus: dem Nasenweg, der Luftröhre, den Hauptstammbronchien, den Segmentbronchien, den leitenden Atemwegen, den Respirationswegen und den Luftbläschen.

13. Verfahren nach Anspruch 12, wobei der proximale Teil ein Hauptstammbronchus ist, und der distale Teil einer ist, der ausgewählt wird aus der Gruppe bestehend aus einer 4. bis 8. Verzweigungsstruktur.

14. Verfahren nach Anspruch 9, wobei das Abgrenzen mindestens eines ersten Teils des Atems, eines zweiten Teils des Atems und eines dritten Teils des Atems umfasst:
Abgrenzen eines vierten Teils des Atems und eines fünften Teils des Atems oder
Bestimmen einer Dauer mindestens eines Teils einer Ausatmungsphase und Teilen der Dauer in Zeitabschnitte, die dem ersten Teil des Atems, dem zweiten Teil des Atems und dem dritten Teil des Atems entsprechen, oder
Bestimmen von Charakteristiken im Atemsensorsignal, wobei die Charakteristiken ausgewählt werden aus der Gruppe bestehend aus: einer Nullsignalamplitude, einer Spitzensignalamplitude, einem Nulldurchgang von einem negativen Wert zu einem positiven Wert, einem Nulldurchgang von einem positiven Wert zu einem negativen Wert, einem Plateau in der Signalamplitude, einer Änderung der Steilheit der Signalamplitude, einem Nullpunkt der Differenz des Signals, einer Spitze der Differenz des Signals, einem Nullpunkt der zweiten Differenz des Signals, einer Spitze der zweiten Differenz des Signals, einem Nullpunkt einer Transformierten des Signals, einer Spitze einer Transformierten des Signals.

15. Verfahren nach Anspruch 11, ferner umfassend:
a) Benachrichtigen eines Patienten, dass er mit einer gewünschten Frequenz atmen soll; und
Verifizieren, dass der Patient mit der gewünschten Frequenz atmet, wobei das Bestimmen, ob ein Atemmusterparameter des Atems das Atemmusterparameterkriterium erfüllt, ein Sammeln des Atems nach einer Verifizierung umfasst, dass der Patient mit einer gewünschten Frequenz atmet; oder
b) Messen des Stickoxids in mindestens zwei Teilen des Atems; und
Korrigieren der Konzentration des in einem physiologisch gültigen Teil gemessenen Stickoxids mit dem in einem anderen Teil gemessenen Stickoxid; oder
c) Messen des Analyten in mehreren Atemzügen zum Bestimmen eines zusammengesetzten Endwerts.

## Revendications

1. Appareil pour analyser des niveaux d'oxyde nitrique dans la respiration, comprenant :
une canule d'échantillonnage nasal (1) ;
un capteur de respiration pour mesurer un paramètre de schéma respiratoire ;
un système d'échantillonnage de gaz respiratoire comprenant une pluralité de vannes (V1-V7) pour isoler des sections du système d'échantillonnage de gaz respiratoire ;
une pompe (12) en aval d'au moins deux de la pluralité de vannes (V1-V7) qui aspire une pluralité de respirations d'un patient dans la canule d'échantillonnage nasale (1) et à travers le système d'échantillonnage de respiration ;
un premier processeur contenant des instructions exécutables pour analyser les paramètres du schéma respiratoire de la pluralité de respirations ;
un deuxième processeur contenant des instructions exécutables pour établir en temps réel, sur la base des paramètres de schéma respiratoire analysés de la pluralité de respirations, des critères de paramètre de schéma respiratoire qui délimitent une respiration physiologiquement représentative et une respiration physiologiquement non représentative ;
un troisième processeur contenant des instructions exécutables pour déterminer si un paramètre de schéma respiratoire d'une respiration, expirée après la pluralité de respirations, répond aux critères de paramètre de schéma respiratoire ;
un quatrième processeur contenant des instructions exécutables pour, en réponse à une détermination qu'un paramètre de schéma respiratoire de la respiration satisfait les critères de paramètre de schéma respiratoire, délimiter au moins une première section de la respiration, une deuxième section de la respiration, et une troisième section de respiration, la première section de respiration étant expirée avant la deuxième section de respiration et la deuxième section de respiration étant expirée avant la troisième section de la respiration ;
un cinquième processeur contenant des instructions exécutables pour commander la pluralité de vannes (V1-V7) afin d'isoler la première section de respiration, la deuxième section de respiration et la troisième section de respiration les unes des autres ; et
un analyseur (14) pour analyser les niveaux d'oxyde nitrique dans au moins une de la deuxième section de la respiration et de la troisième section de la respiration séparément de l'autre.

2. Appareil selon la revendication 1, les instructions exécutables pour commander la pluralité de vannes comprenant des instructions exécutables pour rejeter la première section de respiration.

3. Appareil selon la revendication 1, les premier, deuxième, troisième, quatrième et cinquième processeurs comprenant un seul processeur.

4. Appareil selon la revendication 1, les critères de paramètre de schéma respiratoire comprenant une mesure d'un paramètre de stabilité de la fréquence respiratoire en fonction du temps et une mesure d'un paramètre de fréquence respiratoire.

5. Appareil selon la revendication 1, la deuxième section de respiration correspondant à une section de l'arbre broncho-pulmonaire entre une section proximale et une section distale, ou la deuxième section de respiration correspondant à une section de l'arbre des voies respiratoires choisie dans le groupe constitué par : les voies aériennes nasales, la trachée, les bronches de souche principale, les bronches segmentaires, les voies aériennes conductrices, les voies aériennes respiratoires et les alvéoles.

6. Appareil selon la revendication 5, la section proximale étant une bronche de souche principale et la section distale étant une structure sélectionnée dans le groupe consistant en une 4ème ou 8ème structure de ramification.

7. Appareil selon la revendication 1, les instructions exécutables pour délimiter au moins une première section de la respiration, une deuxième section de la respiration et une troisième section de la respiration comprenant des instructions exécutables pour délimiter une quatrième section de la respiration et une cinquième section de la respiration, ou comprenant des instructions exécutables pour déterminer une durée d'au moins une partie d'une phase d'expiration et diviser la durée en sections temporelles correspondant à la première section de la respiration, à la deuxième section de la respiration et à la troisième section de la respiration, ou comprenant des instructions exécutables pour déterminer des caractéristiques dans le signal du capteur de respiration, les caractéristiques étant sélectionnées dans le groupe constitué de : une amplitude de signal nulle, une amplitude de signal de pointe, un passage par zéro d'une valeur négative à une valeur positive, un passage par zéro d'une valeur positive à une valeur négative, un plateau dans l'amplitude du signal, un changement de pente de l'amplitude du signal, un zéro du différentiel du signal, une pointe du différentiel du signal, un zéro du second différentiel du signal, une pointe du second différentiel du signal, un zéro d'une transformée du signal, une pointe d'une transformée du signal.

8. Appareil selon la revendication 1, comprenant en outre :
a) un sixième processeur comprenant des instructions exécutables pour notifier à un patient de respirer à une fréquence souhaitée ; et un septième processeur comprenant des instructions exécutables pour vérifier que le patient respire à la fréquence souhaitée, les instructions exécutables pour déterminer si un paramètre de schéma respiratoire de la respiration satisfait aux critères de paramètre de schéma respiratoire comprenant des instructions exécutables pour collecter la respiration après une vérification que le patient respire à une fréquence souhaitée ;
b) un huitième processeur comprenant des instructions exécutables pour mesurer l'oxyde nitrique dans au moins deux sections de la respiration et corriger le niveau d'oxyde nitrique mesuré dans une section physiologiquement valide avec l'oxyde nitrique mesuré dans une autre section ; ou
c) un neuvième processeur comprenant des instructions exécutables pour mesurer l'analyte dans plusieurs respirations afin de déterminer une valeur de composition finale.

9. Procédé d'analyse des niveaux d'oxyde nitrique dans la respiration, comprenant :
l'aspiration d'une pluralité de respirations d'un patient respirant normalement en utilisant une canule d'échantillonnage nasale (1), une pompe (12) étant utilisée pour aspirer la pluralité de respirations ;
l'analyse de paramètres du schéma respiratoire de la pluralité de respirations ;
l'établissement en temps réel, sur la base des paramètres de schéma respiratoire analysés de la pluralité de respirations, de critères de paramètre de schéma respiratoire qui délimitent une respiration physiologiquement représentative et une respiration physiologiquement non représentative ;
la détermination si un paramètre de schéma respiratoire d'une respiration, expirée après la pluralité de respirations, satisfait aux critères de paramètre de schéma respiratoire ;
en réponse à une détermination qu'un paramètre de schéma respiratoire de la respiration satisfait aux critères de paramètre de schéma respiratoire, la délimitation d'au moins une première section de la respiration, d'une deuxième section de la respiration, et d'une troisième section de la respiration, la première section de respiration étant expirée avant la deuxième section de respiration et la deuxième section de respiration étant expirée avant la troisième section de respiration ;
la commande d'une pluralité de vannes (V1-V7) pour isoler la première section de respiration, la deuxième section de respiration et la troisième section de respiration les unes des autres ; et
l'analyse de niveaux d'oxyde nitrique dans au moins une de la deuxième section de la respiration et de la troisième section de la respiration séparément de l'autre.

10. Procédé selon la revendication 9, la commande de la pluralité de valves comprenant le rejet de la première section de respiration.

11. Procédé selon la revendication 9, les critères de paramètre de schéma respiratoire comprenant une mesure d'un paramètre de stabilité de la fréquence respiratoire en fonction du temps et une mesure d'un paramètre de fréquence respiratoire.

12. Procédé selon la revendication 9, la deuxième section de respiration correspondant à une section de l'arbre broncho-pulmonaire entre une section proximale et une section distale, ou la deuxième section de respiration correspondant à une section de l'arbre des voies respiratoires choisie dans le groupe constitué par : les voies aériennes nasales, la trachée, les bronches de souche principale, les bronches segmentaires, les voies aériennes conductrices, les voies aériennes respiratoires et les alvéoles.

13. Procédé selon la revendication 12, la section proximale étant une bronche de souche principale et la section distale étant une structure choisie dans le groupe constitué par une 4ème à une 8ème structure de ramification.

14. Procédé selon la revendication 9, la délimitation d'au moins une première section de la respiration, d'une deuxième section de la respiration et d'une troisième section de la respiration comprenant :
la délimitation d'une quatrième section de la respiration et d'une cinquième section de la respiration, ou
la détermination d'une durée d'au moins une partie d'une phase d'expiration et la division de la durée en sections temporelles correspondant à la première section de la respiration, à la deuxième section de la respiration et à la troisième section de la respiration, ou
la détermination de caractéristiques dans le signal du capteur de respiration, les caractéristiques étant choisies dans le groupe constitué par : une amplitude de signal nulle, une amplitude de signal de pointe, un passage par zéro d'une valeur négative à une valeur positive, un passage par zéro d'une valeur positive à une valeur négative, un plateau dans l'amplitude du signal, un changement de pente de l'amplitude du signal, un zéro du différentiel du signal, une pointe du différentiel du signal, un zéro du second différentiel du signal, une pointe du second différentiel du signal, un zéro d'une transformée du signal, une pointe d'une transformée du signal.

15. Procédé selon la revendication 11, comprenant en outre :
a) la notification à un patient de respirer à une fréquence souhaitée ; et
la vérification que le patient respire à la fréquence souhaitée, la détermination si un paramètre de schéma respiratoire de la respiration satisfait les critères de paramètre de schéma respiratoire comprenant la collecte de la respiration après une vérification que le patient respire à une fréquence souhaitée ; ou
b) la mesure de l'oxyde nitrique dans au moins deux sections de la respiration ; et
la correction du niveau d'oxyde nitrique mesuré dans une section physiologiquement valide avec l'oxyde nitrique mesuré dans une autre section ; ou
c) la mesure de l'analyte dans plusieurs respirations pour déterminer une valeur de composition finale.
